(19) Europäisches Patentamt
European Patent Office
Office européen des brevets

(11) **EP 1 961 407 B1**

(12) **EUROPÄISCHE PATENTSCHRIFT**

(45) Veröffentlichungstag und Bekanntmachung des Hinweises auf die Patenterteilung:
**02.12.2009 Patentblatt 2009/49**

(51) Int Cl.:
***A61K 6/04*** *(2006.01)*

(21) Anmeldenummer: **07102905.2**

(22) Anmeldetag: **22.02.2007**

(54) **Aufbrennfähige, niedrigschmelzende Nickel-Chrom-Legierung zur Herstellung keramisch verblendeter Dentalrestaurationen**

Fireable, low melting nickel-chrome alloy for manufacturing ceramic lined dental restorations

Alliage nickel-chrome à cuire pour la fabrication de restauration dentaire revêtue de céramique

(84) Benannte Vertragsstaaten:
**AT BE BG CH CY CZ DE DK EE ES FI FR GB GR HU IE IS IT LI LT LU LV MC NL PL PT RO SE SI SK TR**

(43) Veröffentlichungstag der Anmeldung:
**27.08.2008 Patentblatt 2008/35**

(73) Patentinhaber: **BEGO Bremer Goldschlägerei Wilh.-Herbst GmbH & Co KG**
**28359 Bremen (DE)**

(72) Erfinder: **Abend, Ulrich**
**28790, Schwanewede (DE)**

(74) Vertreter: **Eisenführ, Speiser & Partner Patentanwälte Rechtsanwälte**
**Postfach 10 60 78**
**28060 Bremen (DE)**

(56) Entgegenhaltungen:
**EP-A1- 0 275 843        WO-A-99/37825**
**DE-A1- 3 309 481       DE-C1- 3 214 490**
**FR-A1- 2 378 869       US-A- 4 243 412**
**US-A- 4 249 943**

• **DATABASE WPI Week 199435 Derwent Publications Ltd., London, GB; AN 1994-284387 XP002443181 & RU 2 009 243 C1 (VOLKOV V A) 15. März 1994 (1994-03-15)**
• **DATABASE WPI Week 198140 Derwent Publications Ltd., London, GB; AN 1981-72409D XP002443182 & JP 56 102540 A (SANKIN KOGYO KK) 17. August 1981 (1981-08-17)**
• **DATABASE WPI Week 199629 Derwent Publications Ltd., London, GB; AN 1996-285554 XP002443183 & RU 2 048 574 C1 (KUGUSHIN A A) 20. November 1995 (1995-11-20)**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die Erfindung betrifft eine aufbrennfähige Legierung zur Herstellung keramisch verblendeter Dentalrestaurationen, beispielsweise für die Herstellung von Kronen, Brücken, Einlagen und anderen Zahnprothesen, die mit einer Keramikoberfläche versehen werden sollen, sowie eine entsprechende verblendete Dentalrestauration.

Bei der erfindungsgemäßen Legierung handelt es sich um eine edelmetallfreie, korrosionsbeständige Legierung auf Nickel-Chrom-Basis (NiCr-Basis), bei der vorzugsweise auf die Verwendung von bekannt toxischen Elementen, insbesondere von Beryllium, verzichtet wird.

Legierungen auf Nickel-Chrom-Basis sind beispielsweise aus den folgenden Veröffentlichungen bekannt:

DE 24 32 014 C2, DE 25 28 547 C2, DE 27 13 755 A1, DE 32 14 490 C1, DE 35 40 323 A1, DE 36 09 132 C2, DE 36 30 321 A1, EP 0 275 843 B1, US 2,636,818, US 4,124,381, WO 99/37825.

In RU 2 009 243 C1 werden Legierungen zum Gießen von Zahnersatz der mit Keramik beschichtet ist, offenbart. Die Legierungen enthalten in Gew.-%: C 0,005 - 0,06, Si 1,5 - 2,5, Mn 0,01 - 0,3, Cr 22,0 - 25,0, Mo 9,0 - 11,0, Fe 0,1 - 3,0, Co 0,1 - 4,0, V 0,15 - 0,30 und eines oder mehrere Elemente ausgewählt aus der Gruppe bestehend aus Ce, La, Nd und Pr 0,2 - 1,2 und der Rest ist Nickel. Als wesentlicher Vorteil dieser Legierung wird ein verbesserter Haftverbund mit der Keramik angegeben. Es wird weiter offenbart, dass diese Legierungen leichter zu polieren und zu beschleifen bzw. fräsen sind.

In US 4,243,412 werden Legierungen beschrieben, die in Gew.-% 10 - 20% Chrom, 4 - 10 % Molybdän, 3 - 6% Eisen, 2 - 6% Niob, bis zu 2% Aluminium, 1 - 3% Silizium und 0,05 - 0,5% Kohlenstoff enthalten. Der Rest der Legierung besteht aus Nickel. Es wird die Eignung dieser Legierungen zur Verwendung im Zahnbereich, insbesondere für Zahnersatz mit Porzellan, erwähnt.

Die japanische Offenlegungsschrift JP56-102540 A offenbart Nickellegierungen für den Dentalbereich, die in Gew.-% 3 - 42% Kobalt, 5 - 30% Chrom, 2 - 18% Molybdän, 0 - 10% Wolfram, 0 - 10% Kupfer, 0,1 - 3% Silizium und 0,01 - 0,5% Kohlenstoff enthalten, wobei Nickel den Rest der Legierung ausmacht.

Auch im Handel sind eine Reihe von aufbrennfähigen Legierungen auf NickelChrom-Basis erhältlich. Es sind dabei NiCr-Legierungen weit verbreitet, die unter Verwendung von Beryllium (Be) legiert sind. Diese Legierungen werden von den Zahntechnikern insbesondere wegen ihrer relativ dünn erscheinenden hellgrauen Oxidschicht nach zahntechnischen Temperaturbehandlungen (z. B. "Oxidbrand" oder "keramische Brände") und ihres niedrigen Schmelzintervalls (Liquidustemperatur in der Regel im Bereich von 1250 °C bis 1300°C) geschätzt. Das helle Oxid erleichtert die Arbeitsschritte beim Ausarbeiten für die keramische Verblendung, da sich eine helle dünne Oxidschicht leichter entfernen lässt bzw. selbst bei nicht vollständiger Entfernung visuell nicht negativ auffällt, und auch bei der keramischen Verblendung selbst, da sich eine helle Oxidschicht leichter vollständig durch eine ebenfalls helle, weil zahnfarbene, Verblendkeramik abdecken lässt.

Beryllium erleichtert das Aufschmelzen der Legierung beim zahntechnischen Vergießen. Der große Nachteil entsprechender Legierungen ist allerdings, dass Beryllium als Element aber auch als Bestandteil von Dentallegierungen als toxisch und cancerogen eingestuft ist. Außerdem zeigen solche Legierungen auf Grund ihrer Zusammensetzung ein ungenügendes bis schlechtes Korrosionsverhalten. Daher sind als Alternative seit den 80er bzw. 90er Jahren ds 20. Jahrhunderts viele aufbrennfähige NiCr-Legierungen insbesondere unter dem Aspekt der biologischen Verträglichkeit entwickelt worden, die ohne Beryllium legiert sind. Diese zeigen als Nachteil ein grünes oder sogar fast schwarzes (dunkles) Oxid, welches von Zahntechnikern als negativ empfunden wird, da es sich schwieriger mit der Verblendkeramik abdecken lässt. Die Oxidschicht wird auch häufig als dicker empfunden als bei berylliumhaltigen Legierungen. Auch zeigen diese Legierungen in der Regel ein Schmelzintervall, welches um ca. 100 °C über dem von berylliumhaltigen NiCr-Legierungen liegt. Das höhere Schmelzintervall beding eine relativ hohe Vorwärmtemperatur (in der Regel mindestens 900°C) für die Muffeln, in welche die flüssige Legierung gegossen wird. Daraus können Passungsprobleme resultieren. Für eine möglichst gute Passung wäre ein vergleichsweise niedriges Schmelzintervall von unter 1350 °C vorteilhaft, wie es die berylliumhaltigen Legierungen in der Regel aufweisen, und außerdem die Möglichkeit der Einstellung einer vergleichsweise niedrigen Vorwärmtemperatur von unter 900°C, wie es die berylliumhaltigen Legierungen teilweise erlauben.

Aufbrennfähige Nickel-Chrom-Legierungen werden in der Praxis häufig mit Keramiken verblendet, deren linearer Wärmeausdehnungskoeffizient (WAK-Wert) im Bereich von etwa 12-14 $[10^{-6} \text{ K}^{-1}]$ liegt, ermittelt im Temperaturbereich von 25 - 500 °C. Auch die erfindungsgemäße aufbrennfähige Legierung ist zur Verblendung mit derartigen Verblendkeramiken vorgesehen.

Bei der Konzeption neuer aufbrennfähiger Legierungen (auf Nickel-Chrom-Basis) muss der Fachmann eine Vielzahl technischer Eigenschaften berücksichtigen und versuchen, ausgewählte Eigenschaften besonders günstig einzustellen, ohne dabei die anderen Eigenschaften in besonders nachteiliger Weise zu beeinflussen.

Im Zusammenhang mit der Verwendung üblicher Legierungen auf Nickel-Chrom-Basis wurde es bislang von den Fachleuten häufig als nachteilig empfunden, dass der richtige Gießzeitpunkt nicht mit ausreichender Sicherheit erkannt werden kann; deshalb werden bereits in größerem Umfang automatische Gießzeitpunkt-Erkennungssysteme als Hilfsmittel eingesetzt. Einige Gießsysteme (z. B. solche mit Widerstandsheizung oder solche mit Erwärmung unter Verwendung von Sauerstoff-Erdgas-Brennern) sind darüber hinaus nicht in der Lage, die Legierungen auf Temperaturen von deutlich über 1400 °C zu erhitzen.

Die gegossene Legierung darf beim Erstarren nach dem Gießen nicht zur Bildung von Warmrissen neigen. Dazu ist es wichtig, dass das Schmelzintervall (=Temperaturdifferenz zwischen erstem Schmelzen der Legierung ("Solidustemperatur") und dem vollständigen Aufschmelzen ("Liquidustemperatur")) nicht zu breit ist, weil das eine Erstarrung mit ungleichmäßiger Verteilung der einzelnen Legierungsbestandteile im Legierungsgefüge (= "Seigerungen") zur Folge haben könnte. Weiterhin ist es wichtig, dass die mechanischen Eigenschaften, wie Dehngrenze, Zugfestigkeit und Bruchdehnung, die im Zugversuch ermittelt werden, in einem ausgewogenen Verhältnis stehen, wobei insbesondere die Bruchdehnung nicht zu kleine Werte aufweisen darf. Die angestrebte Ausgewogenheit der mechanischen Eigenschaften wird insbesondere dann regelmäßig nicht erreicht, wenn es beim Erstarren der Legierung zu Ausscheidungen von harten Sprödphasen, wie z. B. Carbiden, Nitriden oder Boriden, kommt, welche besonders ungünstig sind, wenn sie an den Korngrenzen als großvolumige Gebilde auftreten.

Eine weitere für den Zahntechniker wesentliche Eigenschaft ist eine gute Bearbeitbarkeit der Oberfläche, was einerseits mit spanabhebenden Werkzeugen (=Ausarbeiten) und anderseits mit die Oberfläche glättenden Werkzeugen (=Polieren) durchgeführt wird. Hierbei hat es sich als zweckmäßig erwiesen, wenn die Härte der Legierung, gemessen als Vickers-Härte (HV10), nicht über 300 HV10, vorzugsweise nicht über 260 HV10 und bevorzugt im Bereich von 180 bis 260 HV10 liegt.

Ferner wird seitens der Hersteller von aufbrennfähigen NiCr-Legierungen bzw. damit kompatiblen Verblendkeramiken regelmäßig eine Verfahrensgestaltung empfohlen, bei der vor dem Durchführen des eigentlichen Keramikauftrages ein Brand zur Konditionierung der Legierungsoberfläche durchgeführt wird, der teilweise als Oxidbrand, teilweise als Kontrollbrand oder als "Degassing" ausgestaltet wird. Diese Brandführung soll eine ausreichende Haftgrundlage für die Verblendkeramik schaffen. Im Falle des Kontrollbrandes dient dieser lediglich zu einer Beurteilung der Legierungsoberfläche. Eine einheitliche Oxidfarbe wird dann als Indikator dafür angesehen, dass die Metalloberfläche eine einheitliche Zusammensetzung aufweist. Entmischungen oder Fehlstellen wie z.B. Lunker oder Porositäten würden anhand einer anderen Einfärbung der entsprechenden Stelle leicht identifizierbar. Die beim Kontrollbrand entstandene Oxidschicht wird in der Regel vor dem keramischen Verblenden wieder entfernt, z. B. durch Abstrahlen.

Andere Hersteller empfehlen für ihre NiCr-Legierungen eine Verfahrensgestaltung, bei der das keramisch verblendete Gerüst (=Dentalrestauration) nach den keramischen Bränden einer Langzeitabkühlung oder einer Temperphase unterzogen wird, um den WAK der Keramik in geeigneter Weise zu steuern. Der WAK der meisten handelsüblichen Dentalkeramiken mit "normaler Expansion" (WAK-Wert im Bereich von etwa 12 bis 14 [$10^{-6}$ $K^{-1}$] im Temperaturbereich von 25 - 500 °C) erhöht sich nämlich (auf Grund des Wachstums von Leuzitkristallen), wenn die Abkühlphase durch Langzeitabkühlung oder durch Tempern zeitlich gestreckt wird. In der Praxis gilt es als günstig, wenn der WAK der Keramik etwas niedriger ist als der WAK der zu verblendenden aufbrennfähigen Legierung. Auf diese Weise wird die Dentalkeramik nach dem Abkühlen überwiegend unter Druckspannungen gesetzt. Druckspannungen sind für keramische Materialien weit weniger kritisch als Zugspannungen, welche schon in geringem Ausmaß zu Sprüngen in der Keramikschicht oder sogar zu Abplatzungen von größeren Keramiksplittern führen können. Ursache hierfür ist, dass keramische Materialien generell eine deutlich höhere Druckfestigkeit im Vergleich zur Zugfestigkeit aufweisen. Sind die Wärmeausdehnungskoeffizienten der Legierung und der Dentalkeramik somit nicht optimal aufeinander abgestimmt, kommt es zu den soeben beschriebenen Sprüngen oder Abplatzungen in der Keramik. Wird jedoch eine Langzeitabkühlung oder eine Temperphase durchgeführt, um den WAK-Wert der Keramik besser auf den der Legierung abzustimmen, so beinhaltet dies den Nachteil, dass der Zahntechniker eine vergleichsweise lange Zeit bis zum nächsten Bearbeitungsschritt warten muss. Pro Brennzyklus dauert eine Langzeitabkühlung bzw. eine Temperphase etwa 3 bis 10 Minuten länger als eine "normale" Abkühlung.

Das Durchführen eines (zusätzlichen) Oxidbrands oder Kontrollbrands wird ebenfalls als nachteilig angesehen, da es einen zusätzlichen Arbeitsschritt bedeutet, der etwa 5 bis 10 Minuten erfordert.

Angesichts der vorstehend beschriebenen Nachteile bei der Verwendung bekannter NiCr-Legierungen war es die der vorliegenden Erfindung zu Grunde liegende Aufgabe, eine NiCr-Legierung anzugeben, bei der

(i) das Oxid in den unterschiedlichen Bearbeitungsschritten möglichst hell und keinesfalls dunkelgrün oder schwarz aussehen sollte,

(ii) das Schmelzintervall möglichst niedrig (möglichst unter 1350 °C oder noch besser unter 1300 °C) liegen sollte,

(iii) der Gießzeitpunkt in allen gängigen dentalen Gießsystemen eindeutig erkennbar ist,

(iv) eine Vorwärmtemperatur von unter 900°C, idealerweise von ca. 800°C eingestellt werden kann und/oder

(v) es nach dem Aufbrennen einer üblichen Verblendkeramik nicht erforderlich ist, eine Langzeitabkühlung oder ein Tempern durchzuführen.

Die Einstellung der Eigenschaften sollte dabei vorzugsweise ohne Verwenden des toxischen und cancerogenen Elementes Beryllium erreicht werden.

Dabei sollten die sonstigen für eine aufbrennfähige Dentallegierung wichtigen Legierungsmerkmale innerhalb der vom Fachmann bevorzugten Bereiche liegen.

Erfindungsgemäß wird diese Aufgabe gelöst durch eine aufbrennfähige Legierung zur Herstellung keramisch verblendeter Dentalrestaurationen, bestehend aus:

- Nickel 37 oder mehr Gew.-%,

- Chrom 18 bis 23,5 Gew.-%,

- Molybdän, Wolfram insgesamt 8 bis 16 Gew.-%,

- entweder

 (i) Silicium 0,5 bis 3,4 Gew.-% und
 Bor 0,31 bis 2,5 Gew.-%,
 oder

 (ii) Silicium 2,1 bis 3,4 Gew.-% und
 Bor 0 bis 0,05 Gew.-%,

- Niob 0 bis 0,9 Gew.-%,

- Mangan 0 bis 0,5 Gew.-%,

- Kohlenstoff 0 bis 0,02 Gew.-%,

- Eisen, Cobalt, Kupfer insgesamt 0 bis 30 Gew.-%,

- Aluminium, Titan, Zirkonium, Hafnium, Yttrium, Lanthan, Cer, sonstige Seltenerdmetalle, Calcium, Strontium insgesamt 0 bis 0,5 Gew.-%,

- Beryllium 0 bis 0,3 Gew.-%

- sonstige Metalle, Halbmetalle und weitere Bestandteile insgesamt 0 bis 10 Gew.-%,

wobei sich die Gewichtsprozentangaben jeweils auf das Gesamtgewicht der Legierung beziehen.

Vorzugsweise umfasst die erfindungsgemäße Legierung kein Beryllium.

Die erfindungsgemäße aufbrennfähige Legierung umfasst einen Anteil von 37 oder mehr, vorzugsweise 37 bis 75 Gewichtsprozent (Gew.-%) Nickel (Ni). Es hat sich gezeigt, dass ein Anteil an Nickel von über 75 Gew.-% zu einer unerwünscht reduzierten Festigkeit und durch die einhergehende Erniedrigung des Chrom- und/oder Molybdänanteils zu einer Verringerung der Korrosionsfestigkeit führen würde.

Vorzugsweise wird im Rahmen der Erfindung eine Verringerung des Nickel-Gehaltes unter 55 Gew.-% durch eine Erhöhung des Gehaltes an Eisen, Cobalt und/oder Kupfer ausgeglichen, so dass die erfindungsgemäße Legierung vorzugsweise insgesamt 55 oder mehr Gew.-% an Nickel, Cobalt, Kupfer und Eisen umfasst.

Vorzugsweise umfasst eine erfindungsgemäße Legierung 60 bis 70 Gewichtsprozent, bevorzugt 62 bis 67 Gewichtsprozent Nickel, insbesondere sofern die Gesamtmenge an Eisen, Cobalt und Kupfer 5 Gew.-% oder weniger beträgt.

In der erfindungsgemäßen Legierung beträgt der Anteil an Chrom (Cr) 18 bis 23,5 Gewichtsprozent. Es hat sich gezeigt, dass ein Chrom-Anteil von weniger als 18 Gewichtsprozent zu einer inakzeptabel hohen Korrosionsanfälligkeit der entsprechenden Legierung und somit zu deren Löslichkeit in der Mundhöhle führt. Ein Anteil von mehr als 23,5 Gewichtsprozent führt hingegen zu einer Legierung, die - mit steigendem Chromanteil zunehmend - ein unerwünscht dunkles Oxid aufzeigt. Bereits ein Chromanteil von 24 Gewichtsprozent hat sich hinsichtlich der Oxidfarbe schon als nicht mehr akzeptabel gezeigt. Die Abhängigkeit der Oxidfarbe vom Chromanteil ist aus dem Stand der Technik nicht bekannt.

Vorzugsweise liegt der Anteil an Chrom in der erfindungsgemäßen Legierung (insbesondere bei einer, die 60 bis 70 Gew.-% Nickel und gegebenenfalls maximal 5 Gew.-% an Eisen, Cobalt und Kupfer enthält, siehe oben) im Bereich von 19 bis 23 Gewichtsprozent, besonders bevorzugt im Bereich von 21,5 bis 22,5 Gewichtsprozent, insbesondere wenn primär eine optimale Korrosionsresistenz angestrebt ist und eine leicht dunklere Oxidfarbe dafür in Kauf genommen wird.

Die erfindungsgemäße Legierung enthält Molybdän (Mo) und Wolfram (W) in einer Gesamtmenge im Bereich von 8 bis 16 Gewichtsprozent. In diesem Bereich wird ein guter Kompromiss zwischen Korrosionsfestigkeit und mechanischen Eigenschaften wie Härte, Festigkeit, Sprödigkeit und Wärmeausdehnungskoeffizient gefunden.

Ein Gesamtanteil an Molybdän und/oder Wolfram von weniger als 8 Gewichtsprozent führt - mit abnehmendem Molybdän-/Wolfram-Gehalt zunehmend - (i) zu einer inakzeptabel hohen Korrosionsanfälligkeit der entsprechenden Legierung insbesondere im sauren Milieu, welches im Mund insbesondere in Spalten ("Spaltkorrosion") auftritt, und daher auch (ii) zu deren Löslichkeit in der Mundhöhle. Ein Gesamtanteil von mehr als 16 Gewichtsprozent führt hingegen zu einer Legierung, die durch vermehrtes Auftreten von einer Ausscheidungsphase zu einer starken Versprödung der Legierung führt.

Vorzugsweise liegt der Anteil an Molybdän und/oder Wolfram in der erfindungsgemäßen Legierung im Bereich von 9 bis 12 Gewichtsprozent, besonders bevorzugt im Bereich von 9,5 bis 11 Gewichtsprozent, insbesondere wenn primär eine optimale Korrosionsresistenz angestrebt ist.

Unter Berücksichtigung des minimalen Anteils an Chrom in einer erfindungsgemäßen Legierung ergibt sich unter Berücksichtigung des Mindestgehaltes an Molybdän bzw. Wolfram das folgende Ergebnis für die "Wirksumme", die als Bestandteil einschlägiger Normen zum Abschätzen des Korrosionsverhaltens allgemein akzeptiert ist:

$$[Cr] + 3,3 \, (0,5 * [W] + [Mo]) \geq 31,2.$$

Der angegebene Minimalwert für die erfindungsgemäße Legierung liegt über dem gemäß DIN 13912 geforderten Wert für die Wirksumme von 30.

Dies zeigt in Verbindung mit durchgeführten Korrosionsuntersuchungen, dass sich innerhalb der gesamten Spanne der angegebenen Zusammensetzung eine hohe Korrosionsresistenz erreichen lässt.

Bevorzugt ist eine erfindungsgemäße Legierung, deren Wert für die Wirksumme bei mindestens 50 liegt. Ein solcher Wert ist nur erzielbar, wenn die Legierung Molybdän enthält.

Innerhalb des genannten Bereiches sind Molybdän und Wolfram gegeneinander austauschbar. Wie oben ausgeführt, lassen sich gemäß der Formel für die Wirksumme günstige Korrosionseigenschaften durchaus auch mit einer molybdänfreien Legierung erreichen. Besonders günstig im Hinblick auf die Korrosionseigenschaften ist es jedoch, wenn die Legierung Molybdän enthält. Bevorzugt ist der Einsatz von Molybdän, da sich damit im Sinne einer möglichst hohen Wirksumme eine bessere Korrosionsresistenz erreichen lässt.

Bevorzugt ist eine erfindungsgemäße Legierung, wobei der Gewichtsanteil von Molybdän größer ist als der von Wolfram, vorzugsweise zumindest doppelt so groß.

Der Anteil an Silicium (Si) in einer erfindungsgemäßen Legierung liegt im Bereich von 0,5 bis 3,4 Gewichtsprozent, wenn die Legierung Bor in einem Anteil von 0,31 bis 2,5 Gew.-% enthält, bzw. im Bereich von 2,1 bis 3,4 Gewichtsprozent für eine Zusammensetzung ohne bzw. fast ohne Bor. In den angegebenen Mengenbereichen trägt Silicium insbesondere zu einem niedrigen Schmelzintervall und zu einer ausreichend niedrigen Viskosität der Schmelze bei, ohne die Korrosionsfestigkeit nennenswert zu reduzieren oder die Legierung durch vermehrtes Auftreten einer Ausscheidungsphase ("Silicid" - Ausscheidungen) stark zu verspröden. Innerhalb der genannten Bereiche wirkt das Silicium darüber hinaus in gewünschter Weise als Sauerstofffänger. Überdies lässt sich mit einer erfindungsgemäßen Legierung auf Grund des erfindungsgemäß vorhandenen Anteils an Silicium ein günstiger Haftverbund zu üblichen Verblendkeramiken herstellen.

Ein Anteil an Silicium von mehr als 3,4 Gewichtsprozent würde hingegen - mit steigendem Siliciumanteil zunehmend - zu einer verstärkten Versprödung des fertigen Produktes (Gussstückes) und beim zahntechnischen Guss zur Bildung von Oxidschichten auf der Schmelze führen, welche insbesondere in Gießgeräten mit induktiver Erwärmung an Luft (Normalatmosphäre) das Erkennen des richtigen Gießzeitpunktes erschweren würde. Bei sehr viel höheren als den erfindungsgemäß vorhandenen Anteilen an Silicium würde sich zudem ein vermehrt zweiphasiges Gefüge (mit "Silicid"-Ausscheidungen) ausbilden, was eine sehr starke Zunahme der Sprödigkeit der Legierung zur Folge hätte.

Der Einsatz von weniger als 0,5 Gewichtsprozent Silicium (bei Anwesenheit von 0,31 bis 2,5 Gew.-% Bor) bzw. von weniger als 2,1 Gewichtsprozent Silicium (bei zumindest nahezu vollständiger Abwesenheit von Bor, siehe oben) würde nicht mehr in ausreichendem Maße zu den oben genannten gewünschten Effekten (Erniedrigung des Schmelzintervalls, Erniedrigung der Viskosität der Schmelze) führen.

Vorzugsweise liegt der Anteil an Silicium in einer erfindungsgemäßen Legierung im Bereich von 1,0 bis 2,9 Gewichtsprozent und bevorzugt von 1,0 bis 2,7 Gewichtsprozent (bei Anwesenheit von 0,31 bis 2,5 Gew.-% Bor) bzw. von 2,1 bis 2,9 Gewichtsprozent (bei (weitgehender) Abwesenheit von Bor, siehe oben), besonders bevorzugt zwischen 1,6 und 2,4 Gew.-% bzw. 2,1 und 2,4 Gew.-% ("mit" bzw. "ohne" Bor), wenn primär möglichst günstige Korrosionseigenschaften und eine nicht zu hohe Härte bzw. eine günstige mechanische Bearbeitbarkeit ("Fräsen", Ausarbeiten, Polieren) angestrebt ist.

Bevorzugt ist eine erfindungsgemäße Legierung (insbesondere in einer der als bevorzugt gekennzeichneten Ausgestaltungen), die gemäß Alternative (i) 0,31 bis 2,5 Gewichtsprozent Bor umfasst.

Eigene Untersuchungen haben gezeigt, dass auch Bor (B) - ganz ähnlich wie Silicium - die Viskosität der Schmelze (Ausfließverhalten) und das Schmelzintervall sowie weiterhin das Schmelzverhalten (Gießzeitpunkterkennung) im positiven Sinne beeinflussen kann. Allerdings ist durch die eigenen Versuche auch sehr deutlich geworden, dass der Gehalt an Bor genau kontrolliert werden sollte, da der Borgehalt außer auf das Schmelzverhalten auch auf die mechanischen Eigenschaften einen beachtlichen Einfluss aufweist. Der Anteil an Bor in einer erfindungsgemäßen Legierung muss entweder im Sinne einer Verunreinigung bzw. eines Spurenbestandteils höchstens 0,05 Gew.-% betragen, d.h. unterhalb der Löslichkeitsgrenze von Bor in der Legierung (d.h. auch niedriger als für andere Spurenbestandteile / Verunreinigungen üblich, für die allgemein eine Grenze höchstens 0,1 Gew.-% akzeptiert wird), so dass es nicht zu Ausscheidungen von "Borid"-Phasen kommt, oder aber (und dies ist bevorzugt) im Bereich von 0,31 bis 2,5 Gewichtsprozent. In diesem höheren Mengenbereich trägt Bor - ähnlich wie Silicium - sehr wirksam zu einem niedrigen Schmelzintervall und zu einer niedrigen Viskosität der Schmelze bei, ohne bereits die Korrosionsfestigkeit nennenswert zu reduzieren oder die Legierung durch zu starkes Auftreten von Ausscheidungsphasen zu sehr zu verspröden. Innerhalb dieses Bereiches wirkt das Bor darüber hinaus in gewünschter Weise als "Aufheller" für das während des zahntechnischen Gießprozesses gebildete Oxid und das bei den keramischen Brennprozessen gebildete Oxid. Überdies lässt sich in dem höheren Bor-Mengenbereich mit einer erfindungsgemäßen Legierung auf Grund des vorhandenen Anteils an Bor ein günstiger Haftverbund zu üblichen Verblendkeramiken herstellen, so dass dann ein etwas niedrigerer Siliciumgehalt in der erfindungsgemäßen Zusammensetzung möglich ist.

Erfindungsgemäß wird bei einem Siliciumanteil von 0,5 bis < 2,1 Gewichtsprozent Bor im genannten höheren Mengenbereich (0,31 bis 2,5 Gew.-%) eingesetzt, so dass Schmelzintervall, Viskosität (Ausfließverhalten) und Schmelzverhalten (Gießzeitpunkterkennung) positiv beeinflusst werden und Bor zudem als Aufheller wirkt.

Ein Anteil an Bor von mehr als 2,5 Gewichtsprozent würde hingegen zu einer inakzeptabel hohen Sprödigkeit des fertigen Produktes (Gussstückes) führen. Bei diesen hohen Borgehalten bildet sich in erhöhtem Maße ein zweiphasiges Gefüge (mit "Borid"-Ausscheidungen) aus, was die oben erwähnte Versprödung der Legierung zur Folge hat und auch das Polieren der Oberfläche erschwert.

Ein Einsatz von Bor in einer Menge im (Zwischen-) Bereich von > 0,05 bis < 0,31 Gewichtsprozent würde nicht in ausreichendem Maße zu den oben genannten gewünschten Effekten (Erniedrigung des Schmelzintervalls, Erniedrigung der Viskosität der Schmelze, Aufhellen des Oxids) führen. In eigenen Versuchen zeigte sich, dass ein solcher Borgehalt im Bereich von > 0,05 bis < 0,31 Gew.-% in einer entsprechenden Legierung lediglich zu einem sehr breiten Schmelzintervall (teilweise bis zu 150°C) führt, was unerwünschte Seigerungseffekte (ungleichmäßige Verteilung der Legierungsbestandteile innerhalb der verschiedenen Gefügebestandteile) zur Folge hat.

Vorzugsweise liegt der Anteil an Bor in einer erfindungsgemäßen Legierung im Bereich von 0,35 bis 1,5 Gew.-% (insbesondere bei einem Siliciumgehalt in bevorzugten Bereich von 1,0 bis 2,9 Gew.-%), bevorzugt im Bereich von 0,4 bis 1,0 Gew.-% und besonders bevorzugt im Bereich von 0,51 bis 0,9 Gew.-% (insbesondere bei einem Siliciumgehalt zwischen 1,6 und 2,4 Gew.-%), oder aber alternativ im Sinne einer Verunreinigung / eines Spurenbestandteiles im Bereich von 0 bis 0,05 Gew.-%, wobei in letzterem Fall die Legierung vorzugsweise völlig frei von Bor ist.

Eine bevorzugte erfindungsgemäße Legierung umfasst entweder

(i) Silicium     1,0 bis 2,9 Gew.-% und
Bor       0,35 bis 1,5 Gew.-%,
oder

(ii) Silicium     2,1 bis 2,9 Gew.-% und
Bor       0 bis 0,05 Gew.-%.

Bevorzugte erfindungsgemäße Legierungen besitzen eine Vickershärte (HV10) von maximal 260 HV10. Eine Härte im Bereich von 180 bis 220 HV10 ist dabei besonders bevorzugt. Um diese Härte zu erreichen, ist es besonders wichtig, insbesondere den Silicium- und den Bor-Anteil in den oben beschriebenen bevorzugten Bereichen einzustellen.

Der Anteil an Mangan (Mn) in einer erfindungsgemäßen Legierung liegt im Bereich vom 0 bis 0,5 Gewichtsprozent. Mangan (sofern vorhanden) fungiert in einer erfindungsgemäßen Legierung zusätzlich zum Silicium als Sauerstofffänger, als Haftoxidbildner und als Entschwefelungsmittel. Dadurch trägt Mangan dazu bei, dass die Viskosität einer Schmelze einer erfindungsgemäßen Legierung entsprechend niedrig ist. Darüber hinaus schützt es in gewissem Umfang das Silicium, indem es bei ausreichend hoher Konzentration bevorzugt mit Sauerstoff reagiert. Das Silicium kann somit in erster Linie seinen Hauptfunktionen (Schmelzpunkterniedrigung und niedrige Viskosität der Schmelze) gerecht werden.

Ein Anteil an Mangan von mehr als 0,5 Gewichtsprozent ist nicht notwendig, während ein deutlich höherer Gehalt (z.B. über 1 Gewichtsprozent) die Oxidfarbe negativ beeinflussen würde und auch beim Schmelzen in gängigen Schmelzgeräten in unerwünschter Weise mit dem Tiegelmaterial reagieren würde, was die Standzeit der Tiegel deutlich verringern würde und daher in starkem Maße unerwünscht ist.

Unterhalb eines Anteils an Mangan von 0,1 Gewichtsprozent werden im Einzelfall die vorstehend genannten Funktionen des Mangans in einer entsprechenden Legierung nicht mehr zur vollen Zufriedenheit erreicht. Für viele Anwendungen bedeutet dies allerdings keine wesentliche Einschränkung. Dies gilt insbesondere unter der Voraussetzung einer sauberen Verarbeitung unter ausschließlicher Verwendung von Neumaterial. Dies ist auf den relativ hohen Siliciumgehalt in den erfindungsgemäßen Legierungen zurückzuführen.

Vorzugsweise liegt der Anteil an Mangan in einer erfindungsgemäßen Legierung im Bereich von 0,1 bis 0,5 Gewichtsprozent, besonders bevorzugt im Bereich von 0,1 bis 0,3 Gewichtsprozent.

Der Anteil an Niob (Nb) in einer erfindungsgemäßen Legierung liegt im Bereich von 0 bis 0,9 Gewichtsprozent. Niob (sofern vorhanden) fungiert in einer erfindungsgemäßen Legierung als "Stabilisator", indem es nicht zu vermeidende Kohlenstoffverunreinigungen, die entweder in der Legierung vorhanden sind oder durch die zahntechnischen Bearbeitungsschritte eingebracht werden, in Form von unschädlichen Niobcarbiden bindet und so verhindert, dass sich unerwünschte Carbidausscheidungen aus anderen Legierungsbestandteilen bilden. Carbide, z. B. von Chrom oder Molybdän, könnten eine Versprödung der Legierung oder eine Verschlechterung der Korrosionsresistenz zur Folge haben.

Ein Anteil an Niob von mehr als 0,9 Gewichtsprozent ist für diese Funktion nicht erforderlich, während ein höherer Niobgehalt die Oxidfarbe negativ beeinflussen würde und auch beim Schmelzen in gängigen Schmelzgeräten in unerwünschter Weise mit dem Tiegelmaterial reagieren würde, was die Standzeit der Tiegel deutlich verringern würde.

Ein Anteil an Niob von weniger als 0,1 Gewichtsprozent würde im Einzelfall dazu führen, dass die vorstehend genannten Funktionen des Niobs in einer entsprechenden Legierung nicht mehr zur vollen Zufriedenheit erreicht würden. Ähnlich wie bei Mangan bereits ausgeführt, bedeutet dies allerdings für viele Anwendungen keine wesentliche Einschränkung. Dies gilt insbesondere unter der Voraussetzung einer sauberen Verarbeitung unter ausschließlicher Verwendung von Neumaterial, was auf den relativ hohen Chrom- bzw. Molybdängehalt in den erfindungsgemäßen Legierungen zurückzuführen ist.

Vorzugsweise liegt der Anteil an Niob in einer erfindungsgemäßen Legierung im Bereich von 0,1 bis 0,8 Gewichtsprozent, bevorzugt zwischen 0,2 und 0,6 Gewichtsprozent.

Wie bereits erwähnt, kann die Anwesenheit von Kohlenstoff (C) in einer erfindungsgemäßen Legierung zu unerwünschten Karbidausscheidungen und somit zu einer Versprödung oder einer Verschlechterung der Korrosionsresistenz führen. Bereits ein Kohlenstoffanteil von über 0,02 Gewichtsprozent, beispielsweise 0,05 Gewichtsprozent, hat in eigenen Untersuchungen zu einer Beeinflussung der mechanischen Eigenschaften geführt. Eine erfindungsgemäße Legierung enthält maximal 0,02 Gew.-% Kohlenstoff (als Verunreinigung), vorzugsweise maximal 0,01 Gew.-%, und ist bevorzugt völlig frei von Kohlenstoff.

Die erfindungsgemäße Legierung umfasst in jedem Falle (i) Nickel, (ii) Chrom, (iii) Molybdän und/oder Wolfram und (iv) Silicium. Die Verwendung von Mangan, Niob und Bor hat für viele Anwendungen Vorteile und wird daher prinzipiell bevorzugt. Unter den im vorangegangenen Text beschriebenen Einschränkungen kann aber auch darauf verzichtet werden. Alle anderen Bestandteile sind optional.

Vorzugsweise werden in einer erfindungsgemäßen Legierung mehrere oder alle der vorstehend als bevorzugt gekennzeichneten Ausgestaltungen kombiniert. Besonders bevorzugt ist somit eine erfindungsgemäße Legierung umfassend oder bestehend aus:

- Nickel 60 bis 70 Gew.-%,

- Chrom 19 bis 23 Gew.-%,

- Molybdän, Wolfram insgesamt 9 bis 12 Gew.-%,

- entweder

    (i) Silicium 1,0 bis 2,9 Gew.-% und
    Bor 0,35 bis 1,5 Gew.-%,

oder

(ii) Silicium        2,1 bis 2,9 Gew.-% und
Bor        0 bis 0,05 Gew.-%.

- Niob        0,1 bis 0,8 Gew.-%

- Mangan        0,1 bis 0,5 Gew.-%,.

- Kohlenstoff        0 bis 0,01 Gew.-%.

- Eisen, Cobalt, Kupfer        insgesamt 0 bis 5 Gew.-%,

wobei der Gewichtsanteil von Molybdän größer ist als der von Wolfram, vorzugsweise zumindest doppelt so groß.

In einer erfindungsgemäßen Legierung liegt der Gesamtanteil an Aluminium (Al), Titan (Ti), Zirkonium (Zr), Hafnium (Hf), Yttrium (Y), Lanthan (La), Cer (Ce), sonstigen Seltenerdmetallen, Calcium (Ca) und Strontium (Sr) (entweder als Einzelelement oder als Summe einer Auswahl von mehreren oder allen Mitgliedern dieser Gruppe) im Bereich von 0 bis 0,5 Gewichtsprozent. Die genannte Gruppe umfasst "starke Oxidbildner". Kleinere, wohl dosierte Anteile der genannten Bestandteile in einer erfindungsgemäßen Legierung sind zwar nicht unbedingt notwendig, aber teilweise vorteilhaft, insbesondere in Hinblick auf die Oxidbildung während des Schmelzens. Durch eine richtige Dosierung kann das Schmelzverhalten derart gesteuert werden, dass der richtige Gießzeitpunkt insbesondere in Gießgeräten mit induktiver Erwärmung an Luft für den Zahntechniker durch das Aufreißen der Oxidhaut auf der flüssigen Legierung noch eindeutiger und damit noch leichter erkennbar angezeigt wird. Eine Gesamtmenge an den genannten Verbindungen von mehr als 0,5 Gewichtsprozent würde jedoch regelmäßig zu einem unerwünschten Schmelzverhalten in der Art führen, dass sich beim Schmelzen eine dicke Oxidhaut bildet, die gar nicht aufreißt oder erst dann, wenn die Legierung bereits zu heiß ist.

Vorzugsweise wird gegebenenfalls nur ein Bestandteil ausgewählt aus der genannten Gruppe der "starken Oxidbildner" legiert, und zwar in einem Gehalt von maximal 0,2 Gew.-%. Für ausgewählte Elemente ist der bevorzugte Bereich noch weiter eingeschränkt. So ist für Titan ein Anteil von maximal 0,15 Gew.-% und für Aluminium von maximal 0,1 Gew.-% einzustellen. Vorzugsweise werden in erfindungsgemäßen Legierungen weder Yttrium, Lanthan und Cer noch sonstige Seltenerdmetalle eingesetzt. Bei der Abwesenheit von Seltenerdmetallen kann das Stranggussverfahren eingesetzt werden, während bei ihrer Anwesenheit das technisch und kostenmäßig unvorteilhafte Sauggussverfahren eingesetzt werden muss. Mit dem Einsatz von Yttrium, Lanthan, Cer und sonstigen Seltenerdmetallen ist eine gute Keramikhaftung verbunden. Erfindungsgemäß hat sich jedoch gezeigt, dass eine gute Keramikhaftung auch ohne Seltenerdmetalle erreicht wird, sofern man entweder einen vergleichsweise hohen Anteil von Silicium einsetzt oder aber neben Silicium auch noch Bor (siehe oben).

Sonstige Metalle (z.B. Gallium (Ga), Indium (In), Germanium (Ge), Zinn (Sn), Zink (Zn), Edelmetalle wie die Metalle der Platingruppe, Rhenium (Re), Gold (Au), Silber (Ag)), Halbmetalle und weitere Bestandteile (z.B. Verunreinigungen) können in einer erfindungsgemäßen Legierung in einem Bereich von insgesamt 0 bis 10 Gewichtsprozent vorhanden sein, ohne die Eigenschaften der Legierung nennenswert zu beeinflussen. Darüber hinaus seien insbesondere die Elemente Eisen (Fe), Kobalt (Co), und Kupfer (Cu) erwähnt, die ohne weiteres das Nickel sogar in noch größeren Mengen von bis zu 30 Gew.-% ersetzen können, ohne dass sich die Eigenschaften der Legierung wesentlich ändern würden. Aus Kostengründen wird jedoch insbesondere bei Cobalt lediglich ein Anteil von maximal 5 Gewichtsprozent eingesetzt. Selbst in den erwähnten hohen Anteilen würden die genannten Bestandteile die gewünschten Eigenschaften einer erfindungsgemäßen Legierung nicht wesentlich verändern, z.B. die Oxidfarbe nicht beeinflussen, weil sie entweder ein helles Oxid bilden oder aber so edel sind, dass sie nicht oder nicht wesentlich oxidiert werden. Die Metalle der Platingruppe, Rhenium, Gold und Silber sind in einer erfindungsgemäßen Legierung aus Kostengründen vorzugsweise in einer Menge von insgesamt maximal 0,1 Gewichtsprozent enthalten. Durch Vanadium, Tantal und Stickstoff werden die Eigenschaften einer erfindungsgemäßen Legierung nicht positiv beeinflusst. In geringen Mengen sind sie jedoch tolerierbar, ohne die gewünschten Eigenschaften der erfindungsgemäßen Legierung merklich zu verändern. Vorzugsweise sind daher die genannten Bestandteile in einer erfindungsgemäßen Legierung in einer Menge von insgesamt maximal 0,1 Gewichtsprozent enthalten. Für Vanadium und Tantal sind jedoch auch Gehalte bis maximal 0,5 Gew.-% möglich.

Eine besonders bevorzugte erfindungsgemäße Legierung wie vorstehend definiert (vorzugsweise in einer der vorstehend als bevorzugt bezeichneten Ausgestaltungen) umfasst:

- Vanadium, Tantal        insgesamt 0 bis 0,5 Gew.-%,

- Zirkonium, Hafnium, Yttrium, Lanthan, Cer, sonstige Seltenerdmetalle, Calcium, Strontium     insgesamt 0 bis 0,2 Gew.-%,

- Titan     0 bis 0,15 Gew.-%,

- Aluminium, Stickstoff, Metalle der Platingruppe, Rhenium, Gold, Silber     insgesamt 0 bis 0,1 Gew.-%, und/oder

- weitere Bestandteile     0 bis 1 Gew.-%,     vorzugsweise 0 bis 0,5 Gew.-%,     bevorzugt 0 bis 0,2 Gew.-%.

Bevorzugt ist eine erfindungsgemäße Legierung wie vorstehend definiert, wobei der Gewichtsanteil von Legierungsbestandteilen, soweit diese vorhanden sind, in folgender Reihenfolge abfällt:

Nickel, Chrom, Molybdän, Silicium, Niob, Mangan.

Bevorzugt ist weiterhin eine (insbesondere bevorzugte) Legierung wie vorstehend definiert, nicht umfassend:

Stickstoff, Kohlenstoff, Yttrium, Lanthan, Cer, sonstige Seltenerdmetalle, Vanadium, Tantal, Metalle der Platingruppe, Rhenium, Gold, Silber, Cobalt, Kupfer und/oder Eisen,
da diese Stoffe keinen positiven Einfluss auf die gewünschten Eigenschaften einer erfindungsgemäßen Legierung besitzen.

Ein Beispiel für eine besonders bevorzugte erfindungsgemäße Legierung ist eine Legierung bestehend aus:

- Nickel     62,5 bis 66,5 Gew.-%,

- Chrom     21,5 bis 22,5 Gew.-%,

- Molybdän     9,5 bis 11,0 Gew.-%,

- Silicium     1,9 bis 2,3 Gew.-%,

- Bor     0,6 bis 1,0 Gew.-%,

- Niob     0,2 bis 0,6 Gew.-%,

- Mangan     0,1 bis 0,3 Gew.-% und

- weitere Bestandteile     0 bis 1 Gew.-%,

Eine ganz besonders bevorzugte Ausgestaltung einer erfindungsgemäßen Legierung besitzt die folgende Zusammensetzung:

| | |
|---|---|
| Nickel | 64,5 Gewichtsprozent, |
| Chrom | 22,0 Gewichtsprozent, |
| Molybdän | 10,0 Gewichtsprozent, |
| Silicium | 2,1 Gewichtsprozent, |
| Bor | 0,8 Gewichtsprozent, |
| Niob | 0,4 Gewichtsprozent, |
| Mangan | 0,2 Gewichtsprozent. |

Für diese Legierung wurden folgende Eigenschaften bestimmt:

| | |
|---|---|
| Dichte [g/cm$^3$] | 8,2 |
| Vickershärte [HV10] | 260 |

(fortgesetzt)

| | |
|---|---|
| Elastizitätsmodul [GPa] | ca. 200 |
| Dehngrenze (Rp 0,2) [MPa] | 470 |
| Zugfestigkeit ($R_m$)[MPa] | 880 |
| Bruchdehnung (A5) [%] | 10 |
| Schmelzintervall [°C] | 1200-1280 |
| Gießtemperatur [°C] | ca. 1350 |
| WAK [$10^{-6}K^{-1}$] 25-500 °C | 13,8 |
| WAK [$10^{-6}K^{-1}$] 25-600 °C | 14,1. |

Besonders bevorzugte erfindungsgemäße borfreie Legierungen bestehen aus:

- Nickel         63,3 bis 67,0 Gew.-%,

- Chrom         21,5 bis 22,5 Gew.-%,

- Molybdän         9,5 bis 11,0 Gew.-%,

- Silicium         2,1 bis 2,3 Gew.-%,

- Niob        0,2 bis 0,6 Gew.-%,

- Mangan         0,1 bis 0,3 Gew.-% und

- weitere Bestandteile        0 bis 1 Gew.-%.

Eine ganz besonders bevorzugte Ausführungsform einer erfindungsgemäßen borfreien Legierung besitzt die folgende Zusammensetzung:

| | |
|---|---|
| Nickel | 65,3 Gewichtsprozent, |
| Chrom | 22,0 Gewichtsprozent, |
| Molybdän | 10,0 Gewichtsprozent, |
| Silicium | 2,1 Gewichtsprozent, |
| Niob | 0,4 Gewichtsprozent, |
| Mangan | 0,2 Gewichtsprozent. |

Für diese Legierung wurden folgende Eigenschaften bestimmt:

| | |
|---|---|
| Dichte [g/cm³] | 8,2 |
| Vickershärte [HV 10] | 200 |
| Elastizitätsmodul [GPa] | ca. 200 |
| Dehngrenze ($R_p$ 0,2) [MPa] | 350 |
| Zugfestigkeit ($R_m$) [MPa] | 620 |
| Bruchdehnung (A5) [%] | 30 |
| Schmelzintervall [°C] | 1280-1340 |
| Gießtemperatur [°C] | ca. 1450 |
| WAK [$10^{-6}K^{-1}$] 25-500 °C | 13,8 |
| WAK [$10^{-6}K^{-1}$] 25-600 °C | 14,1. |

Erfindungsgemäße Legierungen, insbesondere in den bevorzugten Ausgestaltungen, und dabei noch etwas stärker ausgeprägt in der borhaltigen Variante (siehe oben) zeichnen sich durch die folgenden Eigenschaften besonders positiv aus, die ohne Verwenden des toxischen und kanzerogenen Elementes Beryllium erreicht wurden:

- helles Oxid, welches in den unterschiedlichen Bearbeitungsschritten je nach Oberflächenzustand (z. B. glatt oder rau) und Behandlungstemperatur hell grünlich oder hell bläulich und teilweise blassgelblich ist und somit nicht die unerwünschten Farben dunkelgrün oder schwarz zeigt;

- ein niedriges Schmelzintervall, das für die borhaltige Variante zwischen 1200 °C und 1280 °C und somit wie gewünscht unter 1300 °C liegt; für die annähernd oder vollständig borfreie Variante (siehe oben) liegt das Schmelzintervall zwischen 1280 °C und 1340 °C und somit im geforderten Bereich von unter 1350 °C;

- günstige Gießzeitpunkterkennung (der Zahntechniker sieht, wann er den Guss auslösen muss); so zeigt z. B. bei vielen untersuchten dentalen Gießgeräten (z. B. Vakuumdruckguss (Nautilus® T, Fa. BEGO) oder Schleuderguss mit induktiver Erwärmung (Fornax® T, Fa. BEGO)) das Aufreißen der Oxidhaut auf der Schmelze eindeutig und für den Zahntechniker leicht erkennbar den richtigen Zeitpunkt zum Auslösen des Gusses an;

- eine Vorwärmtemperatur von 800°C

- keine Langzeitabkühlung und kein Tempern beim keramischen Verblenden erforderlich;

- hohe Passgenauigkeit der aus der Legierung gegossenen Gerüste (Dentalrestaurationen);

- akzeptable Härte, d.h. ausreichend gute Be-/Verarbeitungseigenschaften für die borhaltige Variante; die borfreie Variante weist eine niedrige Härte von 200 HV10 und somit gute Be- und Verarbeitungseigenschaften auf;

- günstige Laserschweißbarkeit;

- hohe Korrosionsresistenz;

- Legierung bildet eine besonders dünnflüssige Schmelze;

- Schmelze der erfindungsgemäßen Legierung besitzt auf Grund der niedrigen Viskosität ein günstiges Ausfließverhalten (daraus resultiert ein hohes Formfüllungsvermögen der Schmelze);

- Legierung ist verblendbar mit allen gängigen Verblendkunststoffen;

- Legierung ist verblendbar mit hoch schmelzenden bzw. normal expandierenden Verblendkeramiken, die einen WAK im Bereich 12-14 [$10^{-6}$K$^{-1}$] (25-500°C) besitzen.

Beim Verblenden mit Keramik besitzt die erfindungsgemäße Legierung die folgenden Vorteile:

- WAK in einem günstigen Bereich für alle handelsübliche Keramikmassen mit normaler Expansion (WAK im Bereich 12-14 [$10^{-6}$ K$^{-1}$]), so dass beim Aufbrennen in der Regel sowohl auf eine Langzeitabkühlung als auch auf ein Tempern verzichtet werden kann;

- alle marktüblichen Keramiken mit einem WAK im Bereich 12-14 [$10^{-6}$ K$^{-1}$] sind verwendbar;

- günstige chemische Haftung (vermutlich deshalb, weil zusätzlich zum Chrom auch Silicium, Bor, sowie, soweit vorhanden, auch Niob und Mangan als Haftoxidbildner wirken);

- hohe Warmfestigkeit (ein Gerüst aus der erfindungsgemäßen Legierung verzieht sich nicht während der Brände).

Vorzugsweise werden bei einer erfindungsgemäßen Legierung mehrere oder alle der obenstehend als bevorzugt bezeichneten Ausgestaltungen miteinander kombiniert.
Die Erfindung betrifft auch eine keramisch verblendete Dentalrestauration, umfassend:

- ein Dentalgerüst aus einer erfindungsgemäßen Legierung sowie

- eine auf das Dentalgerüst aufgebrannte Dentalkeramik mit einem WAK im Bereich von 12-14 [$10^{-6}$K$^{-1}$] (25-500°C).

Weitere Aspekte der Erfindung ergeben sich aus den nachfolgenden, die Erfindung näher beschreibenden Beispielen und den Patentansprüchen.

Beispiele

**Beispiele 1 bis 12:** Erfindungsgemäße Legierungen

|  | 1 | 2 | 3 | 4 | 5 | 6 | 7 | 8 | 9 | 10 | 11 | 12 |
|---|---|---|---|---|---|---|---|---|---|---|---|---|
| Nickel | 37 | 37 | 62 | 62 | 52,2 | 39 | 40,29 | 70 | 63,5 | 61 | 64,5 | 65,3 |
| Chrom | 21 | 21 | 22 | 22 | 18 | 20 | 23,5 | 19,3 | 19 | 23 | 22 | 22 |
| Molybdän | 6,7 | 7 | 11 | 11 | 0,5 | 10 | - | 7 | 9 | 6 | 10 | 10 |
| Wolfram | 9 | 9 | - | - | 8,5 | 4,5 | 16 | 2 | - | 5 | - | - |
| Silicium | 2 | 2 | 2,5 | 2,5 | 2,5 | 2,7 | 3,3 | 1 | 1,2 | 2,8 | 2,1 | 2,1 |
| Bor | 2,2 | 2,2 | 2,4 | 2,5 | 0,05 | 0,49 | - | 0,4 | 1,3 | 1,2 | 0,8 | - |
| Niob | 0,9 | 0,9 | - | - | 0,85 | - | 0,8 | 0,1 | 0,7 | 0,29 | 0,4 | 0,4 |
| Mangan | - | - | - | - | 0,08 | 0,3 | - | 0,1 | 0,2 | 0,1 | 0,2 | 0,2 |
| Kohlenstoff | - | - | - | - | 0,02 | 0,01 | 0,005 | - | - | 0,01 | - | - |
| Eisen | 8,4 | 8,4 | - | - | - | 13 | 10 | - | 2 | - | - | - |
| Cobalt | 7 | 7 | - | - | 5 | - | - | - | 1 | - | - | - |
| Kupfer | - | - | - | - | 12 | 10 | 6 | - | 2 | - | - | - |
| Yttrium | - | - | - | - | 0,2 | - | - | - | - | - | - | - |
| Lanthan | - | - | - | - | - | - | - | - | - | 0,1 | - | - |
| Cer | - | - | - | - | - | - | 0,005 | - | - | - | - | - |
| Aluminium | 0,3 | 0,3 | - | - | 0,05 | - | 0,1 | - | - | - | - | - |
| Calcium | - | - | - | - | - | - | - | - | - | 0,1 | - | - |
| Beryllium | 0,3 | - | 0,1 | - | - | - | - | - | - | - | - | - |
| Vanadium | - | - | - | - | - | - | - | - | - | 0,4 | - | - |
| Tantal | - | - | - | - | - | - | - | - | 0,05 | - | - | - |
| Titan | 0,2 | 0,2 | - | - | - | - | - | 0,1 | - | - | - | - |
| Silber | 5 | 5 | - | - | - | - | - | - | 0,05 | - | - | - |
| Gold | - | - | - | - | 0,05 | - | - | - | - | - | - | - |

Alle Angaben sind Gewichtsprozentangaben, bezogen auf das jeweilige Gesamtgewicht der Legierung.

Alle Legierungen gemäß den Beispielen 1 bis 12 zeigten ein helles Oxid. Eine dunkelgrüne oder schwarze Oxidfarbe wurde bei keiner Legierung beobachtet. Keine der genannten Legierungen wies ein Schmelzintervall von über 1350 °C auf. Der Gießzeitpunkt ließ sich bei der Verwendung der Apparaturen Nautilus® T (Fa. BEGO) sowie Fornax® T (Fa. BEGO) eindeutig erkennen.

**Beispiel 13 (Ausführungsbeispiel):**

13.1 Herstellung eines 6-gliedrigen Brückengerüstes

Es wurde aus Wachs eine 6-gliedrige Oberkiefer-Brücke modelliert; als Modell diente dabei eine reale Patientensituation. Die Mindest-Wandstärke war jeweils 0,3 mm. Es wurden die anatomischen Formen berücksichtigt, so dass der Hauptanteil der Restauration später, nach dem Guss, aus Metall bestand.

Die hergestellte Wachsmodellation wurde in eine phosphatgebundene Einbettmasse eingebettet.

Die resultierende Muffel wurde dann auf eine Temperatur von 800 °C gebracht (Vorwärmtemperatur) und 60 min bei dieser Temperatur gehalten.

Der Guss erfolgte in einer induktionsgeheizten Vakuum-Druck-Gussmaschine (Nautilus® CC+/Fa. BEGO, Programm 191, Gießtemperatur 1370°C) unter Verwendung von Ingots einer erfindungsgemäßen Legierung gemäß Beispiel 11.

Die Ingots wurden in der üblichen Weise erhitzt. Der Guss wurde automatisch ausgelöst. Zum Gießzeitpunkt war die zwischenzeitlich vorhandene Oxidhaut eindeutig aufgerissen.

Nach dem Abkühlen der Muffel wurde die Einbettmasse grob mechanisch entfernt. Danach wurde das erhaltene Brückengerüst mit Korund der Körnung 250 μm (Korox® 250/Fa. BEGO) bei 4 bar abgestrahlt. Abschließend wurde die Oberfläche des Brückengerüstes mit einer feinverzahnten Hartmetallfräse bearbeitet. Aufgrund der akzeptablen Härte und der guten Spanbarkeit der eingesetzten Legierung gestaltete sich das Ausarbeiten einfach für den Zahntechniker.

Die Passung des Gerüstes war im Vergleich mit den Erfahrungen aus dem Abguss anderer NiCr-Legierungen gut.

Anmerkung:  Bei NiCr-Legierungen ist man gewöhnlich ein mehr oder weniger starkes Schaukeln, welches durch Passungsfehler bedingt ist, gewohnt. Dieses war bei insgesamt drei hergestellten 6-gliedrigen Brücken zwei mal sehr gering und einmal nicht zu beobachten. Die Passung der beiden schaukelnden Brücken wurde durch Trennen und Fügen wieder beseitigt. Dazu wurde einmal das Laserschweißen (Zulegematerial: Wiroweld NC/Fa. BEGO) und einmal das Löten (mit Wiron®-Lot/Fa. BEGO) verwendet. Beide Fügetechniken lassen sich problemlos anwenden und sind genauso wie bei den bisher erhältlichen NiCr-Legierungen durchzuführen. Die Festigkeit der Fügestellen wurde in zusätzlichen Versuchen gemäß DIN 13972-2:2002 (Laserschweißbarkeit), bzw. ISO 9333:1990 (Lötung) untersucht. Die Anforderungen der Normen wurden jedes Mal erfüllt bzw. deutlich übertroffen.

13.2 Verblenden des Brückengerüstes mit Dentalkeramik mittels Wash- und Grundmassebrand:

Vor dem keramischen Verblenden wurde die Oberfläche des Brückengerüstes (aus 13.1, siehe oben) nochmals, so wie unter 13.1 beschrieben, abgestrahlt und abgedampft, um die Oberfläche für einen folgenden Washbrand zu konditionieren.

Der Washbrand erfolgte nach Auftrag einer dünnen Suspension (Pasten-Opaker) einer Verblendkeramik des Typs Omega 900 (Fa. Vita). Der Auftrag war dabei nicht deckend.

Es wurde dann, nach Auftrag einer deckenden Schicht Pasten-Opaker des Typs Omega 900 (Fa. Vita), ein Grundmasse-Brand durchgeführt.

Für die Durchführung des Wash- und des Grundmasse-Brandes wurde, soweit hier nicht anders angegeben, gemäß der Verarbeitungsanleitung des Keramik-Herstellers (Vita) verfahren. Es wurden die Temperaturen und Zeiten verwendet, die in der unten stehenden Tabelle angegeben sind. Als Brennofen diente ein Vakumat 300 (Fa. Vita).

Auf eine langsame Abkühlung wurde verzichtet und eine normale (d.h. vergleichsweise schnelle) Abkühlung durchgeführt. Es traten keine Sprünge oder Abplatzungen auf, auch nicht nach längerem Liegenlassen (über 3 Tage). Durch die normale Abkühlung kann der Zahntechniker pro Brand ca. 10 min Zeit einsparen. Der Einsatz der unter 13.1 angegebenen Legierung ermöglicht somit ein sehr ökonomisches Arbeiten.

Im beschriebenen Verfahren wurde auf einen Oxidbrand (950 - 980°C, 5 Minuten mit Vakuum vor dem Washbrand) verzichtet. Ein solcher kann aber ergänzend durchgeführt werden, um die Qualität der Oberfläche zu überprüfen. Im Falle einer ausreichenden Oberflächenqualität dürfen dann keine Schattierungen zu erkennen sein, die Oxidschicht muss vielmehr eine einheitliche Farbe aufweisen. Vor den folgenden Bränden muss die Oxidschicht wieder sorgfältig durch Abstrahlen entfernt werden.

Auf Brände des Typs "Schultermassebrand mit Margin" und "Glanzbrand mit Akzentfluid" (nach dem Grundmassebrand) wurde im Rahmen des Ausführungsbeispieles verzichtet. Solche Brände können aber ergänzend durchgeführt werden.

Gemäß der nachfolgenden Tabelle wurden ergänzend folgende Brände durchgeführt: 1. Dentinbrand, 2. Dentinbrand, Korrekturbrand und Glanzbrand. Dabei wurden wieder Keramikmaterialien des Typs Omega 900 (Fa. Vita) eingesetzt.

Die Verbundfestigkeit wurde in In-vitro-Versuchen (Abschlag -Test, Abschreck-Test und Biegeversuch gemäß DIN EN ISO 9693:2000) bestimmt. Dabei wurden sämtliche Anforderungen deutlich übertroffen.

Die unverblendeten Anteile (Kronenränder, aber auch unverblendete Kronen) ließen sich einfach mit Diamantpolierpaste polieren. Der schnell erreichte Glanz erfüllt die Ansprüche an die Ästhetik und bietet dem Anhaften von z. B. Speiseresten und der Plaquebildung ausreichend großen Widerstand.

Tabelle:

| Brand | Vorwärmtemperatur [°C] | Haltezeit [min] | Heizzeit [min] | Aufheizrate [°C/min] | Endtemperatur [°C] | Haltezeit [min] | Gesamtvakuumzeit [min] |
|---|---|---|---|---|---|---|---|
| Washbrand | 500 | 6 | 6 | 50 | 900 | 3 | 6 |
| Grundmasse | 500 | 6 | 6 | 50 | 900 | 2 | 6 |
| 1. Dentinbrand | 600 | 6 | 6 | 50 | 900 | 1 | 6 |
| 2. Dentinbrand | 600 | 6 | 6 | 50 | 890 | 1 | 6 |
| Korrekturbrand | 600 | 4 | 6 | 33 | 800 | 1 | 6 |
| Glanzbrand | 600 | - | 4 | 75 | 900 | 2 | - |

**Patentansprüche**

1.  Aufbrennfähige Legierung zur Herstellung keramisch verblendeter Dentalrestaurationen, bestehend aus:

    - Nickel          37 oder mehr Gew.-%,
    - Chrom          18 bis 23,5 Gew.-%,
    - Molybdän, Wolfram          insgesamt 8 bis 16 Gew.-%,
    - entweder

        (i) Silicium          0,5 bis 3,4 Gew.-% und
        Bor          0,31 bis 2,5 Gew.-%,
        oder
        (ii) Silicium          2,1 bis 3,4 Gew.-% und
        Bor          0 bis 0,05 Gew.-%,

    - Niob          0 bis 0,9 Gew.-%,
    - Mangan          0 bis 0,5 Gew.-%,
    - Kohlenstoff          0 bis 0,02 Gew.-%,
    - Eisen, Cobalt, Kupfer          insgesamt 0 bis 30 Gew.-%,
    - Aluminium, Titan, Zirkonium, Hafnium, Yttrium, Lanthan, Cer, sonstige Seltenerdmetalle, Calcium, Strontium          insgesamt 0 bis 0,5 Gew.-%,
    - Beryllium          0 bis 0,3 Gew.-%,

    und

    - sonstige Metalle, Halbmetalle und weitere Bestandteile          insgesamt 0 bis 10 Gew.-%,

    wobei sich die Gewichtsprozentangaben jeweils auf das Gesamtgewicht der Legierung beziehen.

2.  Legierung nach Anspruch 1, nicht umfassend Beryllium.

3.  Legierung nach einem der vorangehenden Ansprüche, umfassend:

    - Nickel, Cobalt, Kupfer, Eisen          insgesamt 55 oder mehr Gew.-%.

4.  Legierung nach einem der vorangehenden Ansprüche, umfassend:

    - Nickel          60 bis 70 Gew.-%,
    - Eisen, Cobalt, Kupfer          insgesamt 0 bis 13 Gew.-%, vorzugsweise 0 bis 5 Gew.-%.

5.  Legierung nach einem der vorangehenden Ansprüche, insbesondere nach Anspruch 4, umfassend:

    - Chrom          19 bis 23 Gew.-%, vorzugsweise
    21,5 bis 22,5 Gew.-%.

6.  Legierung nach einem der vorangehenden Ansprüche, umfassend:

    - Molybdän, Wolfram          insgesamt 9 bis 12 Gew.-%, vorzugsweise 9,5 bis 11 Gew.-%.

7.  Legierung nach einem der vorangehenden Ansprüche, wobei gilt:

$$[Cr] + 3,3 (0,5 * [W] + [Mo]) \geq 50$$

8.  Legierung nach einem der vorangehenden Ansprüche, wobei der Gewichtsanteil von Molybdän größer ist als der von Wolfram, vorzugsweise zumindest doppelt so groß.

9. Legierung nach einem der vorangehenden Ansprüche, umfassend:

 - entweder

    (i) Silicium      1,0 bis 2,9 Gew.-% und
    Bor     0,35 bis 1,5 Gew.-%,
    oder
    (ii) Silicium     2,1 bis 2,9 Gew.-% und
    Bor    0 bis 0,05 Gew.-%.

10. Legierung nach einem der vorangehenden Ansprüche, umfassend:

 - Niob    0,1 bis 0,8 Gew.-%.

11. Legierung nach einem der vorangehenden Ansprüche, umfassend:

 - Mangan    0,1 bis 0,5 Gew.-%.

12. Legierung nach einem der vorangehenden Ansprüche, umfassend:

 - Kohlenstoff    0 bis 0,01 Gew.-%.

13. Legierung nach einem der vorangehenden Ansprüche, umfassend oder bestehend aus:

 - Nickel    60 bis 70 Gew.-%,
 - Chrom    19 bis 23 Gew.-%,
 - Molybdän, Wolfram    insgesamt 9 bis 12 Gew.-%,
 - entweder

    (i) Silicium     1,0 bis 2,9 Gew.-% und
    Bor    0,35 bis 1,5 Gew.-%,
    oder
    (ii) Silicium     2,1 bis 2,9 Gew.-% und
    Bor    0 bis 0,05 Gew.-%.

 - Niob    0,1 bis 0,8 Gew.-%
 - Mangan    0,1 bis 0,5 Gew.-%,.
 - Kohlenstoff    0 bis 0,01 Gew.-%.
 - Eisen, Cobalt, Kupfer    insgesamt 0 bis 5 Gew.-%,

 wobei der Gewichtsanteil von Molybdän größer ist als der von Wolfram, vorzugsweise zumindest doppelt so groß.

14. Legierung nach einem der vorangehenden Ansprüche, umfassend:

 - Vanadium, Tantal    insgesamt 0 bis 0,5 Gew.-%,
 - Zirkonium, Hafnium, Yttrium, Lanthan, Cer, sonstige Seltenerdmetalle, Calcium, Strontium    insgesamt 0 bis 0,2 Gew.-%,
 - Titan    0 bis 0,15 Gew.-%,
 - Aluminium, Stickstoff, Metalle der Platingruppe, Rhenium, Gold, Silber    insgesamt 0 bis 0,1 Gew.-%,

 und/oder

 - weitere Bestandteile    0 bis 1 Gew.-%.

15. Legierung nach einem der vorangehenden Ansprüche, wobei der Gewichtsanteil von Legierungsbestandteilen, soweit diese vorhanden sind, in folgender Reihenfolge abfällt:

 Nickel, Chrom, Molybdän, Silicium, Niob, Mangan.

**16.** Legierung nach einem der vorangehenden Ansprüche, wobei gilt: die Vickers-Härte ist maximal 260 HV10.

**17.** Legierung nach einem der vorangehenden Ansprüche, nicht umfassend:

Stickstoff, Kohlenstoff, Yttrium, Lanthan, Cer, sonstige Seltenerdmetalle, Vanadium, Tantal, Metalle der Platingruppe, Rhenium, Gold, Silber, Cobalt, Kupfer und/oder Eisen.

**18.** Legierung nach einem der vorangehenden Ansprüche, bestehend aus:

- Nickel          62,5 bis 66,5 Gew.-%,
- Chrom          21,5 bis 22,5 Gew.-%,
- Molybdän          9,5 bis 11,0 Gew.-%,
- Silicium          1,9 bis 2,3 Gew.-%,
- Bor          0,6 bis 1,0 Gew.-%,
- Niob          0,2 bis 0,6 Gew.-%,
- Mangan          0,1 bis 0,3 Gew.-% und
- weitere Bestandteile          0 bis 1 Gew.-%,

oder

- Nickel          63,3 bis 67,0 Gew.-%,
- Chrom          21,5 bis 22,5 Gew.-%,
- Molybdän          9,5 bis 11,0 Gew.-%,
- Silicium          2,1 bis 2,3 Gew.-%,
- Niob          0,2 bis 0,6 Gew.-%,
- Mangan          0,1 bis 0,3 Gew.-% und
- weitere Bestandteile          0 bis 1 Gew.-%.

**19.** Keramisch verblendete Dentalrestauration, umfassend:

- ein Dentalgerüst aus einer Legierung nach einem der vorangehenden Ansprüche sowie
- eine auf das Dentalgerüst aufgebrannte Dentalkeramik mit einem WAK im Bereich von 12 bis 14 $[10^{-6}K^{-1}]$ (25 - 500 °C).

## Claims

**1.** Veneerable alloy for the production of ceramic-veneered dental restorations, consisting of:

- Nickel          37 wt.% or higher,
- Chromium          18 to 23.5 wt.%,
- Molybdenum, tungsten          in total 8 to 16 wt.%,
- either

(i) Silicon          0.5 to 3.4 wt.% and
Boron          0.31 to 2.5 wt.%, or
(ii) Silicon          2.1 to 3.4 wt.% and
Boron          0 to 0.05 wt.%,

- Niobium          0 to 0.9 wt.%,
- Manganese          0 to 0.5 wt.%,
- Carbon          0 to 0.02 wt.%,
- Iron, cobalt, copper          in total 0 to 30 wt.%.
- Aluminium, titanium, zirconium, hafnium, yttrium, lanthanum, cerium, other rare-earth metals, calcium, strontium          in total 0 to 0.5 wt.%,
- Beryllium          0 to 0.3 wt.%,

and

- Other metals, semimetals and further constituents     in total 0 to 10 wt.%,

wherein the data relating to percentage by weight are each with respect to the total weight of the alloy.

**2.** An alloy according to Claim 1, not comprising beryllium.

**3.** An alloy according to any of the preceding claims, comprising:

- Nickel, cobalt, copper, iron     in total 55 wt.% or higher.

**4.** An alloy according to any of the preceding claims, comprising:

- Nickel     60 to 70 wt.%,
- Iron, cobalt, copper     in total 0 to 13 wt.%, preferably 0 to 5 wt.%.

**5.** An alloy according to any of the preceding claims, in particular according to Claim 4, comprising:

- Chromium     19 to 23 wt.%, preferably 21.5 to 22.5 wt.%,

**6.** An alloy according to any of the preceding claims, comprising:

- Molybdenum, tungsten     in total 9 to 12 wt.%, preferably 9.5 to 11 wt.%.

**7.** An alloy according to any of the preceding claims, wherein:

$$[Cr] + 3.3\,(0.5 \times [W] + [Mo]) \geq 50$$

**8.** An alloy according to any of the preceding claims, wherein the percentage by weight of molybdenum is higher than that of tungsten, preferably at least twice as high.

**9.** An alloy according to any of the preceding claims, comprising:

- either

    (i) Silicon     1.0 to 2.9 wt.% and
    Boron     0.35 to 1.5 wt.%.
    or
    (ii) Silicon     2.1 to 2.9 wt.% and
    Boron     0 to 0.05 wt.%.

**10.** An alloy according to any of the preceding claims, comprising:

- Niobium     0.1 to 0.8 wt.%.

**11.** An alloy according to any of the preceding claims, comprising:

- Manganese     0.1 to 0.5 wt.%.

**12.** An alloy according to any of the preceding claims, comprising:

- Carbon     0 to 0.01 wt.%.

**13.** An alloy according to any of the preceding claims, comprising or consisting of:

- Nickel     60 to 70 wt.%,
- Chromium     19 to 23 wt.°/a,

- Molybdenum, tungsten          in total 9 to 12 wt.%,
- either

    (i) Silicon          1.0 to 2.9 wt.% and
    Boron          0.35 to 1.5 wt.%,
    or
    (ii) Silicon          2.1 to 2.9 wt.% and
    Boron          0 to 0.05 wt.%.

- Niobium          0.1 to 0.8 wt.%,
- Manganese          0.1 to 0.5 wt.%,
- Carbon          0 to 0.01 wt.%,
- Iron, cobalt, copper          in total 0 to 5 wt.%,

wherein the percentage by weight of molybdenum is higher than that of tungsten, preferably at least twice as high.

14. An alloy according to any of the preceding claims, comprising:

- Vanadium, tantalum          in total 0 to 0.5 wt.%,
- Zirconium, hafnium, yttrium, lanthanum, cerium, other rare-earth metals, calcium, strontium          in total 0 to 0.2 wt.%,
- Titanium          0 to 0.15 wt.%,
- Aluminium, nitrogen, metals of the platinum group, rhenium, gold, silver          in total 0 to 0.1 wt.%,

and/or

- further constituents          0 to 1 wt.%.

15. An alloy according to any of the preceding claims, wherein the percentage by weight of alloy constituents, where present, decreases in the following order:

Nickel, chromium, molybdenum, silicon, niobium, manganese.

16. An alloy according to any of the preceding claims, wherein: the Vickers hardness is at most 260 HV10.

17. An alloy according to any of the preceding claims, not comprising:

Nitrogen, carbon, yttrium, lanthanum, cerium, other rare-earth metals, vanadium, tantalum, metals of the platinum group, rhenium, gold, silver, cobalt, copper and/or iron.

18. An alloy according to any of the preceding claims, consisting of:

- Nickel          62.5 to 66.5 wt.%,
- Chromium          21.5 to 22.5 wt.%,
- Molybdenum          9.5 to 11.0 wt.%,
- Silicon          1.9 to 2.3 wt.%,
- Boron          0.6 to 1.0 wt.%,
- Niobium          0.2 to 0.6 wt.%,
- Manganese          0.1 to 0.3 wt.% and
- further constituents          0 to 1 wt.%,

or

- Nickel          63.3 to 67.0 wt.%,
- Chromium          21.5 to 22.5 wt.%,
- Molybdenum          9.5 to 11.0 wt.%,
- Silicon          2.1 to 2.3 wt.%.
- Niobium          0.2 to 0.6 wt.%,

- Manganese       0.1 to 0.3 wt.% and
- further constituents     0 to 1 wt.%.

**19.** Ceramic-veneered dental restoration, comprising:

- a dental framework consisting of an alloy according to any of the preceding claims, and
- a dental ceramic fired onto the dental framework, the dental ceramic having a coefficient of thermal expansion in the range from 12 to 14 [$10^{-6}K^{-1}$] (25 - 500°C).

**Revendications**

**1.** Alliage à cuire, pour la production de restaurations dentaires revêtues de céramique, constitué de :

- nickel     37 % en poids ou plus,
- chrome     18 à 23,5 % en poids,
- molybdène, tungstène    au total, 8 à 16 % en poids,
- soit

    (i) silicium 0,5 à 3,4 % en poids et
    bore     0,31 à 2,5 % en poids,

- soit

    (ii) silicium    2,1 à 3,4 % en poids, et
    bore    0 à 0,05 % en poids,

- niobium     0 à 0,9 % en poids,
- manganèse     0 à 0,5 % en poids,
- carbone     0 à 0,02 % en poids,
- fer, cobalt, cuivre    au total, 0 à 30 % en poids,
- aluminium, titane, zirconium, hafnium, yttrium, lanthane, cérium, autres métaux de terres rares, calcium, strontium    au total, 0 à 0,5 % en poids,
- béryllium     0 à 0,3 % en poids, et
- autres métaux, semi-métaux et autres composants    au total, 0 à 10 % en poids,

les pourcentages en poids se rapportant respectivement au poids total de l'alliage.

**2.** Alliage selon la revendication 1, ne comprenant pas de béryllium.

**3.** Alliage selon l'une des revendications précédentes, comprenant :

- du nickel, cobalt, cuivre, fer    au total, 55 % en poids

ou plus.

**4.** Alliage selon l'une des revendications précédentes, comprenant :

- du nickel     60 à 70 % en poids,
- du fer, cobalt, cuivre au total, 0 à 13 % en poids, de préférence, 0 à 5 % en poids.

**5.** Alliage selon l'une des revendications précédentes, en particulier selon la revendication 4, comprenant :

- du chrome    19 à 23 % en poids, de préférence, 21,5 à 22,5 % en poids.

**6.** Alliage selon l'une des revendications précédentes, comprenant :

- du molybdène, du tungstène au total, 9 à 12 % en poids, de préférence, 9,5 à 11 % en poids.

**7.** Alliage selon l'une des revendications précédentes, correspondant à l'équation :

$$[Cr] + 3,3 (0,5* [W] + [Mo] \geq 50.$$

**8.** Alliage selon l'une des revendications précédentes, dans lequel le pourcentage en poids de molybdène est supérieur à celui du tungstène, de préférence, est au moins le double.

**9.** Alliage selon l'une des revendications précédentes, comprenant :

- soit

    (i) du silicium      1,0 à 2,9 % en poids et
    du bore      0,35 à 1,5 % en poids,

- soit

    (ii) du silicium      2,1 à 2,9 % en poids, et
    du bore      0 à 0,05 % % en poids.

**10.** Alliage selon l'une des revendications précédentes, comprenant :

- du niobium      0,1 à 0,8 % en poids.

**11.** Alliage selon l'une des revendications précédentes, comprenant :

- du manganèse 0,1 à 0,5 % en poids.

**12.** Alliage selon l'une des revendications précédentes, comprenant :

- du carbone      0 à 0,01 % en poids.

**13.** Alliage selon l'une des revendications précédentes, comprenant ou étant constitué de :

- nickel      60 à 70 % en poids,
- chrome      19 à 23 % en poids,
- molybdène, tungstène au total, 9 à 12 % en poids,
- soit

    (i) silicium      1,0 à 2,9 % en poids et
    bore      0,35 à 1,5 % en poids,

- soit

    (ii) silicium      2,1 à 2,9 % en poids, et
    bore      0 à 0,05 % en poids,

- niobium      0,1 à 0,8 % en poids,
- manganèse      0,1 à 0,5 % en poids,
- carbone      0 à 0,01 % en poids,
- fer, cobalt, cuivre      au total, 0 à 5 % en poids,

dans lequel le pourcentage en poids de molybdène est supérieur à celui du tungstène, de préférence est au moins le double.

**14.** Alliage selon l'une des revendications précédentes, comprenant :

- du vanadium, du tantale        au total, 0 à 0,5 % % en poids,
- du zirconium, hafnium, yttrium, lanthane, cérium, autres métaux de terres rares, calcium, strontium        au total, 0 à 0,2 % en poids,
- du titane        0 à 0,15 % en poids,
- de l'aluminium, de l'azote, des métaux du groupe platine, rhénium, or, argent        au total, 0 à 0,1 % en poids et/ou
- d'autres composants        0 à 1 % en poids.

**15.** Alliage selon l'une des revendications précédentes, dans lequel le pourcentage en poids de composants d'alliage, dans la mesure où ceux-ci sont présents, est dans l'ordre suivant :

nickel, chrome, molybdène, silicium, niobium, manganèse.

**16.** Alliage selon l'une des revendications précédentes, dans lequel la dureté Vickers est au maximum de 260 HV10.

**17.** Alliage selon l'une des revendications précédentes, ne comprenant pas de :

azote, carbone, yttrium, lanthane, cérium, autres métaux de terres rares, vanadium, tantale, métaux du groupe platine, rhénium, or, argent, cobalt, cuivre et/ou fer.

**18.** Alliage selon l'une des revendications précédentes, constitué de :

- nickel        62,5 à 66,5 % en poids,
- chrome        21,5 à 22,5 % en poids,
- molybdène        9,5 à 11,0 % en poids,
- silicium        1,9 à 2,3 % en poids,
- bore        0,6 à 1,0 % en poids,
- niobium        0,2 à 0,6 % en poids,
- manganèse        0,1 à 0,3 % en poids et

autres composants        0 à 1 % en poids,
ou

- nickel        63,3 à 67,0 % en poids,
- chrome        21,5 à 22,5 % en poids,
- molybdène        9,5 à 11,0 % en poids,
- silicium        2,1 à 2,3 % en poids,
- niobium        0,2 à 0,6 % en poids,
- manganèse 0,1 à 0,3 % en poids et

autres composants        0 à 1 % en poids.

**19.** Restauration dentaire revêtue de céramique, comprenant :

- une ossature dentaire constituée d'un alliage selon l'une des revendications précédentes, et
- une céramique dentaire cuite sur l'ossature dentaire présentant une valeur WAK de l'ordre de 12 à 14 [$10^{-6}K^{-1}$] (25 à 500° C).

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 2432014 C2
- DE 2528547 C2
- DE 2713755 A1
- DE 3214490 C1
- DE 3540323 A1
- DE 3609132 C2
- DE 3630321 A1
- EP 0275843 B1
- US 2636818 A
- US 4124381 A
- WO 9937825 A
- RU 2009243 C1
- US 4243412 A
- JP 56102540 A